# EUROPEAN PATENT APPLICATION

(11) **EP 2 876 590 A1**
(43) Date of publication of application: **27.05.2015**
(21) Application number: 14193310.1
(22) Date of filing: 14.11.2014
(51) Int. Cl.: G06Q 10/10

(54) **MEDICAL EVENT TRACKING SYSTEM**

(30) Priority: 22.11.2013 US 201314087128
(71) Applicant: Xerox Corporation, Rochester, NY 14644 (US)
(72) Inventor: Csurka, Gabriela, 38920 Crolles (FR); Perronnin, Florent C., 38420 Domene (FR); Jarmasz, Mario Agustin Ricardo, 38240 Meylan (FR)
(74) Representative: Skone James, Robert Edmund

(57) **Abstract**

A medical event tracking system includes memory which stores instructions for detecting new records in a personal health record of a single individual, identifying one of the new records that refers to an event for which an action is to be performed by at least one of the individual and a person acting on behalf of the individual, generating a candidate calendar entry for the identified new record which proposes a time for the action to be performed by the at least one of the individual and the person acting on behalf of the individual, providing for receiving validation of the candidate calendar entry, and providing for sending the validated candidate calendar entry to a personal electronic calendar of at least one of the individual and the person acting on behalf of the individual. A processor in communication with the memory executes the instructions.

## Description

### BACKGROUND

The exemplary embodiment relates to the medical arts and finds particular application in connection with a system and method for organizing personal health-related schedules.

Individuals have to track various sources of health related information throughout their lifetimes, such as doctor appointments, electronic and paper prescriptions, the frequency and times at which medication has to be taken, recurring appointments (such as for blood tests), and electronic and paper medical records. There may also be additional information about medication, treatments and diseases, insurance procedures, employee benefits, and the phone numbers and addresses of doctors and hospitals which a person has to keep track of.

This problem of personal health information management is particularly challenging for patients facing conditions, such as cancer, requiring extended outpatient care. For cancer patients, a primary intervention (such as surgery) is generally followed by therapy (such as radiation, chemotherapy, or hormone therapy) that can last additional weeks, months, or years. As a result, the patient has to be aware of changing treatment regimens while trying to maintain work and home responsibilities.

There have been some attempts to track medical information using personal devices such as tablet computers and cloud computing systems such as Microsoft's Health Vault and Google Health. However, these systems are primarily for storing information and searching it.

Individuals can manually enter appointments and the like in their personal calendars and contact lists. However, this is a time consuming process, particularly for caregivers attending to several sick children or aging adults, and can lead to errors when not updated due to changes or when appointments are forgotten. Thus, it is not easy for individuals to track medical information using the software and devices they use frequently, such as electronic calendars, e-mail, smartphones, tablet computers and PCs.

It would be advantageous to be able to track personal medical information without having to manually enter and re-enter the medical information over time.

### INCORPORATION BY REFERENCE

The following references, the disclosures of which are incorporated herein by reference in their entireties, are mentioned:

U.S. 7,865,386, issued January 4, 2011, entitled APPOINTMENT SCHEDULING SYSTEM, by Shyamal Sarkar.

U.S. Patent No. 8,244,566 issued August 14, 2012, entitled SYSTEMS AND METHODS FOR ON-LINE SCHEDULING OF APPOINTMENTS AND OTHER RESOURCES, by Chad Cooley, et al.

US Pub. No. 20030225597, published December 4, 2003, entitled METHODS AND SYSTEMS FOR THE CREATION AND USE OF MEDICAL INFORMATION, by Joseph H. Levine.

U.S. Pub. No. 20080071543, published March 20, 2008, entitled SECURE PERSONAL HEALTH INFORMATION AND EVENT REMINDER SYSTEM AND PORTABLE ELECTRONIC DEVICE, by Carl T. Jarvis, et al.

U.S. Pub. No. 20120191475, published July 26, 2012, entitled METHOD AND SYSTEM FOR USER CENTRIC, CENTRALIZED ACCESS AND MANAGEMENT OF HEALTHCARE RELATED INFORMATION, DAY TODAY FUNCTIONS AND ADMINISTRATIVE FUNCTIONS ACROSS MULTIPLE PROVIDERS AND ENTITIES IN THE HEALTHCARE ECO-SYSTEM, by Abhishek Pandey.

U.S. Application Serial No. 13/898,805, filed May 21, 2013, entitled TARGETED SUMMARIZATION OF MEDICAL DATA BASED ON IMPLICIT QUERIES by Gabriela Csurka, et al.

### BRIEF DESCRIPTION

In accordance with one aspect of the exemplary embodiment, a medical event tracking system includes memory which stores instructions for detecting new records in a personal health record of a single individual, identifying one of the new records that refers to an event for which an action is to be performed by at least one of the individual and a person acting on behalf of the individual, generating a candidate calendar entry for the identified new record which proposes a time for the action to be performed by the at least one of the individual and the person acting on behalf of the individual, providing for receiving validation of the candidate calendar entry, and providing for sending the validated candidate calendar entry to a personal electronic calendar of at least one of the individual and the person acting on behalf of the individual. A processor in communication with the memory executes the instructions.

In accordance with another aspect of the exemplary embodiment, a medical event tracking method includes, with at least one processor, automatically checking for new records added to a personal health record of an individual, processing at least some of the new records to identify new records that refer to a respective event for which an action is to be performed by at least one of the individual and a person acting on behalf of the individual, for one of the identified new records, generating a candidate calendar entry for the identified new record which proposes a time for the action to be performed by the at least one of the individual and the person acting on behalf of the individual, providing for receiving validation of the candidate calendar entry, and providing for sending the validated candidate calendar entry to a personal electronic calendar of at least one of the individual and the person acting on behalf of the individual.

In accordance with another aspect of the exemplary embodiment, a portable computing device includes a display device, a user input device, a processor, and memory. The memory stores a personal health record of a single individual which includes a set of records pertaining to the health of the individual, a personal electronic calendar for the individual, and instructions. These include instructions for checking for new records which have been added to the personal health record, processing the new records to identify a new record which refers to an event for which an action is to be performed by the individual, generating a candidate calendar entry for the identified new record which proposes a time for the action to be performed by the individual, displaying the candidate calendar entry in a user interface on the display device, providing for validation of the candidate calendar entry by the individual via the user input device, and, if the candidate calendar entry is validated, sending the validated candidate calendar entry to the personal electronic calendar.

### BRIEF DESCRIPTION OF THE DRAWINGS

FIGURE 1 is a functional block diagram of a medical event tracking system in accordance with one aspect of the exemplary embodiment;
FIGURE 2 is a flow chart illustrating a medical event tracking method in accordance with another aspect of the exemplary embodiment;
FIGURE 3 illustrates a user interface generated by the system, in accordance with one embodiment; and
FIGURE 4 illustrates part of the method of FIGURE 2 in one example embodiment.

### DETAILED DESCRIPTION

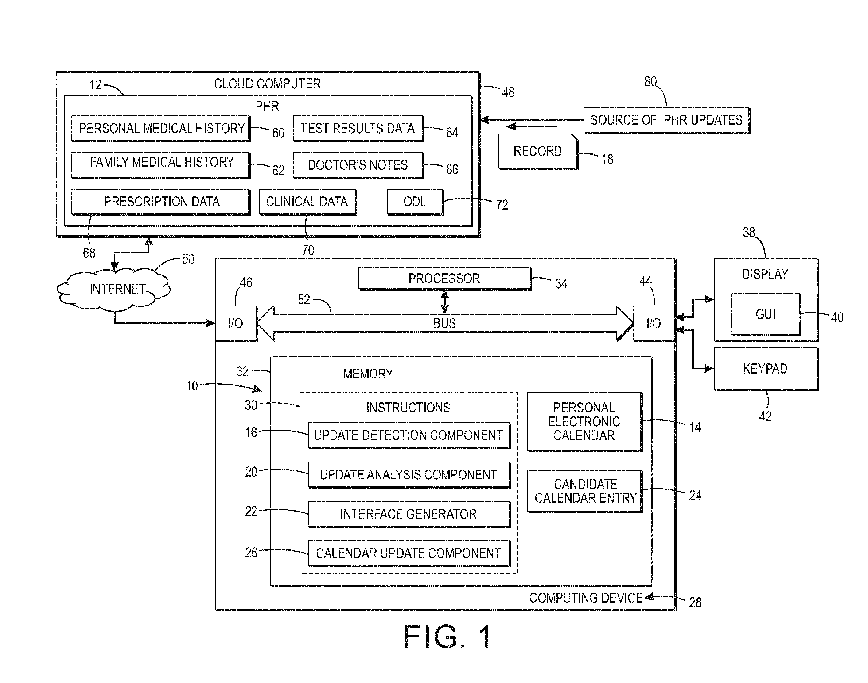

With reference to FIGURE 1, a medical event tracking (MET) system **10** is shown. The exemplary system allows individuals to track various sources of health related information over time, such as scheduling and times of doctor appointments, dates for refilling pharmacy prescriptions, times at which medication is to be taken, scheduling of new or recurring appointments for medical tests, and contact information, where appropriate, which is related to a medical event which is to be or which has been scheduled.

The exemplary system **10** serves as a bridge between an individual person's individual Personal Health Record (PHR) **12** and the person's Personal Electronic Calendar (PEC) **14.** It is assumed that authorization is given to the MET system to access the PHR and communicate with the particular PEC. The system **10** includes several components. In particular, an update detection component **16** detects when new records **18** are added to the PHR **12.** The update component may be configured for checking periodically for new records in the PHR (e.g., every day, hour, or minute), or at other regular or irregular time intervals, or may be automatically notified each time a new record is added. Each new record **18** is one that does not already exist in the PHR **12** or is a modification to an existing record in the PHR.

An update analysis component **20** analyses the textual content of the added records **18** to identify those records which refer to an event, together with any associated temporal information. An event can be anything from which the system may infer that the individual (or another, on behalf of the individual) is expected to take an action, such as to perform a prescribed task, schedule or attend an appointment with a healthcare practitioner or medical test provider, etc. Tasks are actions the individual performs for himself (or with the assistance of a caregiver), such as taking medicines and performing exercises, while appointments involve meetings with other entities, such as doctor's visits and laboratory test appointments.

As used herein, an "individual" (sometimes referred to herein as a patient), refers to the single person to whom the PHR **12** relates, while a "user" can be the individual or a caregiver. The caregiver is a person authorized to act on behalf of the individual in connection with the individual's PHR. Authorization to the caregiver may have been granted by the individual, by a court, or automatically, e.g., by law. A caregiver can be a parent, e.g., in the case of a minor child, other close relative, social worker, nurse making home visits, paid home visitor, or the like. The PEC **14** in which the calendar entries are entered may be that of the individual and/or of an authorized caregiver.

An interface generator **22** generates a candidate calendar entry **24** to be proposed to the user for an event and specified time(s) inferred from the temporal information, if available, to be entered in the PEC **14,** if approved. The interface generator **22** also provides for the user to validate (accept and/or correct), or decline the candidate calendar entry **24.** A calendar update component **26** adds the validated calendar entries to the PEC **14.**

The components **16, 20, 22, 26** may be resident on one or more computing devices, such as the illustrated local (to the user) computing device **28.** Components **16, 20, 22, 26** may be in the form of software instructions **30** that are stored in memory **32** of the computing device **28.** A processor **34,** communicatively connected with the memory **32,** executes the instructions **30.** The system **10** may include a display device **38,** such as a computer monitor or LCD screen, which displays a graphical user interface **40,** and a user input device **42,** such as a keyboard, keypad, touch screen, or the like for receiving user inputs which are communicated to the processor **34** via an interface **44.** In one embodiment, the display device **38** forms part of a mobile computing device **28,** such as a mobile phone. In this case, the display device may be mounted to a casing of the mobile device which houses the processor **34** and memory **32** (which, in some embodiments, may store the PHR **12** and/or software instructions **30).**

In some embodiments, a network interface **46** allows the system **10** to communicate with a remote computing/memory storage device **48,** such as a remote database hosted by one or more cloud computing devices, which stores the user's PHR **12.** The system **10** may then access the PHR **12** via a wired or wireless link **50,** such as the Internet. In other embodiments, the PHR **12** may be stored on the mobile device **28.**

Hardware components **32, 34, 38, 42 44, 46,** of the system may be communicatively linked by a data/control bus **52.**

Example events detected by the system **10** in the records **18** may those that entail actions including performing tasks, such as taking medication, performing health related activities, such as prescribed exercises, and the like, or scheduling or attending an appointment with a healthcare practitioner, medical testing laboratory, or the like. Healthcare practitioners include those qualified to medically treat or provide medical care (including palliative care) to the individual, such as doctors (e.g., surgeons, general practitioners, psychologists, and dentists), registered nurses, physiotherapists, chiropractors, and their staff.

The Personal Health Record (PHR) **12** is an electronic health record which is specific to an individual person. The PHR contains health data and information related to the healthcare of a single patient and is maintained by and accessible to the individual himself/herself (and/or an authorized caregiver of the individual). This is in contrast to health records which are operated by institutions (e.g., hospitals) and which may also contain, for example, billing data to support insurance claims. The purpose of a PHR **12** is to provide a complete and accurate summary of the individual's medical history which may be accessible online. The health data in a PHR may include, for example, records classed in at least some of the following classes: personal medical history **60,** such as allergies and adverse drug reactions, chronic diseases, illnesses, hospitalizations, surgeries and other procedures, and vaccinations; family medical history **62,** such as medical conditions of parents and siblings and causes of death; test results **64,** such as imaging reports (e.g., X-ray) and laboratory test results, such as blood and other body fluid and body tissue test analyses and results from monitoring devices, such as heart rate monitors and blood pressure monitors; narrative doctor notes **66,** e.g., audio and typed recordings made by the individual's healthcare practitioner, prescription data **68,** such as prescribed medications and dosing; clinical data **70,** such as hospital and surgery records; and observations of Daily Living (ODLs) **72,** which are defined by the patient and which may not be specifically requested by a clinician, such as observations about sleep patterns, exercise regimens, food intake, and the like.

The individual's PHR **12** is specific to only a single individual (rather than a group of individuals) and may be stored on a local computing device **28,** such as a mobile computing device, e.g., a smart phone, palmtop or tablet computer, or a desktop device, or the like, that is in the possession of the individual. Additionally or alternatively, the individual's PHR may be stored on a dedicated remote central or distributed database **48** such as Microsoft's HealthVault system, which provides PHR solutions for tablets and PCs. Access to the entire PHR **12** of the individual may be limited to the individual himself or herself, or in the case of a child or incapacitated person, may be accessible to the parent, another relative, or other caregiver who acts on behalf of the individual. The individual may also authorize access to the entire PHR, or to specified parts of it, to a doctor or other healthcare practitioner providing treatment or medical care to the individual. The individual may also authorize the healthcare practitioner and/or their support staff to update the PHR with records **18,** which may be uploaded to the database **48** from a source **80** of PHR updates, such as a hospital, surgery, or pharmacist's computing device, or database linked thereto. As will be appreciated, the PHR **12** may be updated with records from two or more different sources **80.**

All or part of the PHR **12** may be encoded in one or more standardized formats developed for EMRs (Electronic Medical Records), such as HL7, CDA, CCR and XDS, which have been accepted in several countries. The PHR **12** may be accessed through the user interface **40** displayed on the display device **38,** which may follow standard conventions as to how medical information should be displayed so as to make it easier for the individual or caregivers to find information. See, for example, Paul Tang, et al., "Personal Health Records: Definitions, Benefits, and Strategies for Overcoming Barriers to Adoption," JAMIA 13 (2) pp. 121-126 (2006), for a further discussion of PHRs.

The Personal Electronic Calendar (PEC) **14,** which may be stored in memory **32** or in remote memory accessible to the system **12,** may be a conventional calendar application, which assists users in planning and organizing their scheduled appointments and tasks. The user may already have such an application running on a desktop, portable computing device, and/or mobile phone. While in some embodiments, the user may use the electronic calendar to introduce medical appointments manually, in the exemplary embodiment, at least some of the medical events are added to the calendar by the system, after validation by the user.

The computer implemented MET system **10** may be hosted by one or more computing devices, such as a PC, such as a desktop, a laptop, palmtop computer, portable digital assistant (PDA), server computer, mobile phone, tablet computer, combination thereof, or other computing device capable of executing instructions for performing the exemplary method.

The memory **32** may represent any type of non-transitory computer readable medium such as random access memory (RAM), read only memory (ROM), magnetic disk or tape, optical disk, flash memory, or holographic memory. In one embodiment, the memory **32** comprises a combination of random access memory and read only memory. In some embodiments, the processor **34** and memory **32** may be combined in a single chip. Memory **32** stores instructions for performing the exemplary method as well as the processed data.

The input/output (I/O) interface **44, 46** allows the computer to communicate with other devices via a computer network, such as a local area network (LAN) or wide area network (WAN), or the Internet, and may comprise a modulator/demodulator (MODEM) a router, a cable, and and/or Ethernet port.

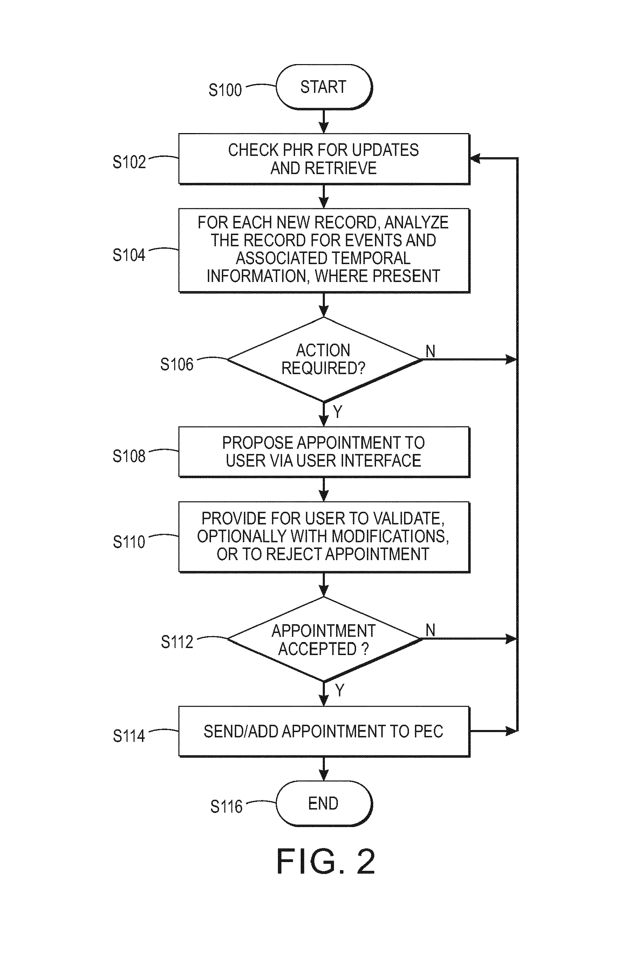

The digital processor **34** can be variously embodied, such as by a single-core processor, a dual-core processor (or more generally by a multiple-core processor), a digital processor and cooperating math coprocessor, a digital controller, or the like. The digital processor **30,** in addition to controlling the operation of the computer **28,** executes instructions stored in memory **32** for performing the method outlined in FIGURE 2. Computer **48** can be similarly configured to computer **28,** with memory and a processor.

As will be appreciated, when parts of the software instructions are distributed over two or more computing devices, such as devices **28, 48,** the processor and memory implementing the instructions for performing the exemplary method can comprise two or more processors and memories.

The term "software," as used herein, is intended to encompass any collection or set of instructions executable by a computer or other digital system so as to configure the computer or other digital system to perform the task that is the intent of the software. The term "software" as used herein is intended to encompass such instructions stored in storage medium such as RAM, a hard disk, optical disk, or so forth, and is also intended to encompass so-called "firmware" that is software stored on a ROM or so forth. Such software may be organized in various ways, and may include software components organized as libraries, Internet-based programs stored on a remote server or so forth, source code, interpretive code, object code, directly executable code, and so forth. It is contemplated that the software may invoke system-level code or calls to other software residing on a server or other location to perform certain functions.

As will be appreciated, FIGURE 1 is a high level functional block diagram of only a portion of the components which are incorporated into a computer system **10.** Since the configuration and operation of programmable computers are well known, they will not be described further.

FIGURE 2 illustrates a method for tracking medical events, which may be performed with the system of FIGURE 1. The method begins at S100.

At S102 an update to an individual's PHR **12** is detected. For example, the update detection component **16** checks regularly for new records in the PHR **12** and retrieves them temporarily into memory **32** accessible to the MET for processing.

At S104, each identified new record **18** is analyzed by the update analysis component **20** to identify events and temporal expressions associated with those events, where present. The analysis component **20** may identify the textual content of the record and process the textual content using natural language processing (NLP) techniques. These techniques may include applying a set of rules configured for identifying whether the new records identify an event (appointment, recurring event, task) that could involve the patient taking an action or actions. The rules may include rules for detection of specific words and phrases, and specified syntactic relations between them, such as subject and object dependencies (relationships between a noun or noun phrase and the syntactically-verb). For example, the rules may be configured to identify an object dependency between *take* and *medicine.* Synonyms and hyponyms of each word in the dependency may be considered as equivalent. For example, Vicodin may be considered equivalent to medicine for the purposes of the example rule. The analysis component **20** also extracts temporal expressions (corresponding to times, dates, and intervals of time) that are associated with each event and from these, determines start (and end) time(s) for a candidate calendar entry specifying the action and its recurrence frequency, if any. The actions may include regular treatments, doctor appointments, etc., as discussed above. The action may be required to be performed by the individual and/or by another person acting on behalf of the individual, such as a caregiver who is authorized to inject the individual with insulin or to schedule a doctor's appointment for the individual.

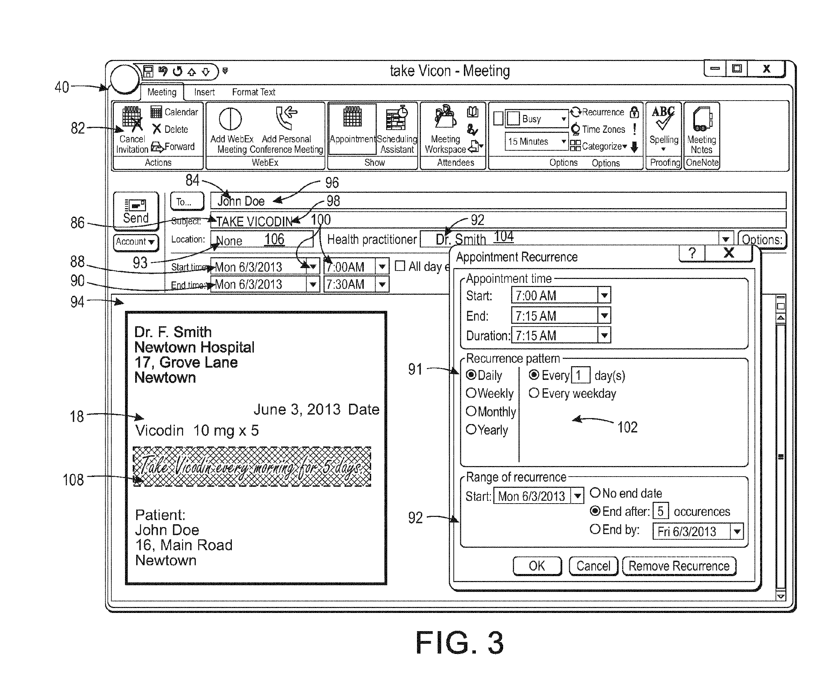

At S106, if the analysis component **20** of the MET system detects an event for which an action is required by or on behalf of the individual, the method proceeds to S108 where the addition of one (or multiple) candidate calendar entries to the PEC **14** is/are proposed to the user, via the user interface **40.** The user interface may display the candidate calendar entry **24** as an appointment request form **82** (e.g., for a meeting, task, or recurring event), as illustrated in FIGURE 3. The form **82** may include some or all of: at least a portion of the original record **18** which was used by the system **10** to suggest the candidate calendar entry **24;** the name of the individual **84,** the identified action **86** to be performed by or on behalf of the individual; proposed start and end times **88, 90** of the action, inferred from the temporal expression; and recurrence information **91,** if appropriate; healthcare practitioner name **92;** and location **93** at which the event is to take place. These may be displayed in respective fields **94, 96, 98, 100, 102, 104, 106** of the form **82.** If no temporal expression is detected, or if it is not specific as to a time, the system may propose a date and/or time, based on the user's PEC and/or on learned user behavior (such as that the user has previously declined to schedule doctor's appointments in a given time period, e.g., between 8 and 9 am, or usually schedules appointments on Monday afternoons).

At S110, provision is for the user to validate or reject the candidate calendar entry **24.** As part of the validation, the user may select to modify the candidate calendar entry, such as by changing the time, if it is not convenient. If at S112, the candidate calendar entry is rejected, the method ends or returns to S102 to await the next PHR update. Otherwise, the method proceeds to S114.

At S114, the validated and optionally modified candidate calendar entry is sent and added to the PEC **14** to as one or more calendar entries. The method ends at S116 or returns to S102.

The method illustrated in FIGURE 2 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use. The computer program product may be integral with the computer **48** and/or **28,** (for example, an internal hard drive of RAM), or may be separate (for example, an external hard drive operatively connected with the computer **18),** or may be separate and accessed via a digital data network such as a local area network (LAN) or the Internet (for example, as a redundant array of inexpensive of independent disks (RAID) or other network server storage that is indirectly accessed by the computer **48** and/or **28,** via a digital network).

Alternatively, the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in FIGURE 2, can be used to implement the method tracking medical events. As will be appreciated, while the steps of the method may all be computer implemented, in some embodiments one or more of the steps may be at least partially performed manually.

Further details of the system and method will now be described.

### Architecture

In one embodiment, the medical event tracking system **10** is a software component of an application which manages the updates to the PHR **12.** In such a case, the MET system **10** can access the PHR data in a secure way. In one embodiment, where a centralized system **48** is used to store the PHRs of multiple users, the MET system **10** may be a software component of this central system. Where the PHR of an individual is stored on his or her mobile device **28,** then the system **10** may run on the mobile device. In other embodiments, the PHR **12** and system **10** may be hosted by separate computing devices **48, 28,** as illustrated, and an encryption component may be used to encrypt the PHR updates **18** which are communicated to the system.

In one embodiment, e.g., when the medical event tracking system **10** and PHR **12** are both hosted by a remote computing device **48,** the PHR **12** may include contact information to send the candidate calendar entries for appointments / reminders / tasks to the user. For example, if the communication between the medical event tracking system **10** and the PEC **14** is performed through emails, this contact information may include one or more email addresses for the user and/or caregiver(s). Since the communicated information is of a sensitive nature, email encryption maybe used to protect privacy.

In another embodiment, the medical event tracking system **10** may connect directly to the user's PEC **14.** For example, the user's PEC may run a medical event tracking client plugin. This embodiment is suited to the case in which the PHR **12,** MET system **10,** and PEC **14** reside on the same computing device **28,** e.g., the user's mobile device.

As will be appreciated, other configurations of computing devices are also contemplated.

### Processing records added to the PHR (S104)

In one embodiment, all records **18** added to the PHR **12** have some searchable text in the form of visible text or metadata, in addition to any non-textual content, such as photographic or X-ray images. The searchable text may comprise temporal information, such as dates, and locations (e.g., when and where the analyses were performed or when the prescriptions were issued), normal health ranges for a patient's vital signs (e.g., blood pressure, glucose level, heart rate, weight, temperature), ASCII transcriptions of handwritten text or voice recordings, etc. If this is not available, different techniques can be applied such as OCR of scanned documents, handwriting recognition, voice to text transcription, and so forth, to generate searchable text. The records **18** may be manually and/or or automatically generated. As an example, the PHR may be updated using wellness devices (e.g., a heart rate monitor), which may subsequently be used to trigger an event in the MET system **10,** e.g., when the results are outside a normal range.

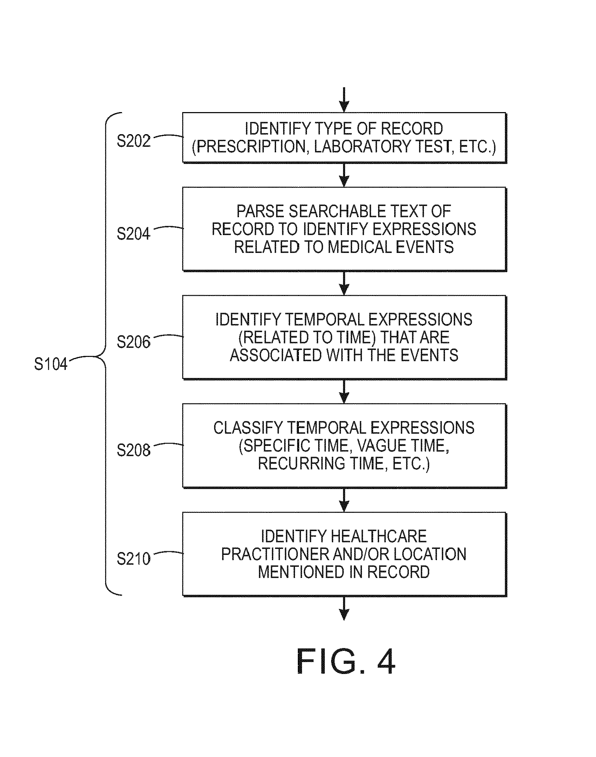

FIGURE 4 illustrates processing of the record (S104) in one embodiment.

At S202, metadata identifying the type of record may be extracted, such as prescription, laboratory, test, doctor's notes, etc. and the record may be labeled with one of a finite set of record types.

At S204, the record is parsed to identify expressions relating to medical events. Depending on the type of record identified at S202, different rules may be applicable for processing the record. The identified event may be converted into a form suited to displaying to the user which identifies the action they are to perform. In some embodiments, identification of an event may employ two or more records. For example, a record which identifies the date of birth of the individual, may be combined with other information, such as a record which indicates the individual's close relative died of a specified medical condition, such as cancer or heart attack, close to that age may be combined to generate an event that the individual should be screened for the condition, based on their age.

At S206, temporal expressions relating to the events are identified, where possible.

At S208, the identified temporal expressions, if any, may be assigned to one of a finite set of types, such as specific time, vague time, recurring time, etc. The assignment may be performed by applying a set of rules which are configured for identifying the various types of temporal expressions or with a classifier which has been trained to classify the temporal expressions according to type. As will be appreciated, the step of identification of temporal expressions may take place before the identification of events.

At S210, additional information, such as the name of the healthcare professional, location of the facility where the event is to occur, etc., may be extracted.

### Parsing text

In one exemplary embodiment, the text is first processed at S204 with a natural language parser, e.g., an incremental parser, such as the Xerox Incremental parser (XIP), which may be integrated in or called on by the analysis component **20.** Examples of such parsers are described, for example, in U.S. Patent No. 7,058,567 by Aït-Mokhtar, et al.; Salah Aït-Mokhtar, et al., "Robustness beyond Shallowness: Incremental Deep Parsing," Natural Language Engineering 8 (2002) 121-144; Aït-Mokhtar, et al., "Incremental Finite-State Parsing," Proceedings of Applied Natural Language Processing, Washington, April 1997; and Aït-Mokhtar, et al., "Subject and Object Dependency Extraction Using Finite-State Transducers," Proceedings ACL'97 Workshop on Information Extraction and the Building of Lexical Semantic Resources for NLP Applications, Madrid, July 1997; Eugene Charniak, "A Maximum-Entropy-Inspired Parser," Proc. NAACL-2000; and the Charniak parser which is available at ftp://ftp.cs.brown.edu/pub/nlparser/, the disclosures of which are incorporated herein by reference. Further details on deep syntactic parsing which may be applied herein are provided in U.S. Pub. No. 20070179776, by Segond, et al., U.S. Pub. No. 20090204596, by Brun, et al., the disclosures of which are incorporated herein by reference.

During parsing of the document, the parser annotates the text strings of the document with tags (labels) which correspond to grammar rules, such as lexical rules, syntactic rules, and dependency (semantic) rules. The lexical rules define relationships between words by the order in which they may occur or the spaces between them. Syntactic rules describe the grammatical relationships between the words, such as noun-verb, adjective-noun. Semantic rules include rules for extracting dependencies (subject-verb relationships, object-verb relationships, etc.), and co-reference links. The application of the rules may proceed incrementally, with the option to return to an earlier rule when further information is acquired.

The natural language parser treats each sentence as a sequence of tokens such as words and punctuation. Each sentence is broken down into a sequence of tokens by the parser. Morphological information is then associated with each token, such as a part of speech, selected from a predefined set of part of speech tags. Dependencies between tokens or groups of tokens (chunks) are then identified. Named Entities may also be tagged by a Named Entity Detector, particularly those which are associated with a medical-related title of a person, organization, or place, such as "Dr. Smith," "Newtown Hospital," "Newtown Pharmacy," and the like.

To facilitate extracting events from medical records (S204), a system as described in above-mentioned U.S. Application Serial No. 13/898,805 may be employed. In particular, the update analysis component 20 (or the PHR update application) may include a transformation component which transforms the health records of the patient into representations in a multidimensional search space, using a predefined ontology which includes a set of medical concepts that are linked by links which show their interrelationships, such as that Vicodin is a type of drug. Then, rules for extraction of expressions involving medical concepts (such as UMLS concepts), such as "take the drug" can be built that rely on such an ontology to allow, for example, tagging of Vicodin with the concept drug. When applied to the record, the generalized rule can recognize "take Vicodin" as satisfying the generic expression for such an event.

The types of syntactic/semantic dependency relationships identified may depend on the specific parser employed and the rules that it applies. As will be appreciated, the parser rules which perform the parsing may be implemented in any suitable order.

The rules for extraction of events and temporal expressions can be written on top of the conventional parser rules.

Different types of records may be treated in different ways. The following illustrate examples and corresponding actions which may be proposed to be added to the calendar via a candidate calendar entry.

### A. Processing records with temporal expressions

For each record **18,** temporal expressions are identified within the actual content and interpreted (S206). Temporal expressions refer to dates, times, and/or frequencies. Examples include "June 16, 2014," "in March," "Next Monday," "every day," "in the morning," "before breakfast," "after eating," "twice daily," "after 4pm," "before bedtime," and combinations thereof. These temporal expressions can be normalized, with respect, for example, to the date of the record, to generate a normalized time entry which specifies the date and optionally also the time, or a range of times, such as Monday June 16, 2014, from 9-5 pm.

There are several methods for identification of temporal expressions and rules which can be applied to normalize them. See, for example, David Ahn, et al., "A Cascaded Machine Learning Approach to Interpreting Temporal Expressions," NAACL-HLT 2007*,* (hereinafter, Ahn, 2007); Caroline Hagège, et al., "Linguistic and Temporal Processing for Discovering Hospital Acquired Infection from Patient Records," Proc. KR4HC, ECAI 2010; Lisa Ferro, et al., "TIDES 2003 standard for the annotation of temporal expressions," Mitre Corp, 2003; Inderjeet Mani, et al., "Robust temporal processing of news," Proc. 38th ACL Annual Meeting, 2000; U.S. Patent Application Serial No. 13/920,462, filed June 18, 2013, entitled COMBINING TEMPORAL PROCESSING AND TEXTUAL ENTAILMENT TO DETECT TEMPORALLY ANCHORED EVENTS, by Caroline Hagège, et al., and Caroline Hagège, et al., "XTM: A robust temporal processor," CICLing Conference on Intelligent Text Processing and Computational Linguistics, Haifa, Israel, February 17-23, 2008, the disclosures of which are incorporated herein by reference.

Temporal expressions may be identified based on the at least partially parsed text, such as dates, times, durations, recurrences, and other normalizable temporal expressions. As examples, temporal expressions such as in "take Vicodin *three times a day for 10 days,"* "need to come back for checkup *in three months,"* "the first echography should be carried out *at 10 to 14 weeks of gestation"* etc., are classified into different classes such as duration, recurrences, generic duration, given dates, and generic or vague points. The temporal expressions are normalized so that they are expressed in a recognized format, such as the Gregorian calendar. Those which do not express a specific date and/or time are normalized with respect to an anchor point such as a date/time on which the report was issued or received by the PHR. The report is then labeled with the normalized temporal expressions.

The labels applied by the parser may be in the form of tags, e.g., XML tags, metadata, log files, or the like.

The parser may attempt to identify relationships in which the temporal expression is an argument of a predicate involving a medically-related action. For example, a sentence such as *take the medicine every morning for five days,* the parser recognizes *take* as being a predicate which is classed as an action and which has as one of its arguments *the medicine* and as two other arguments, two temporal expressions, *every morning* and *for five days.* From this, the analysis component may extract an event comprising an action *take the medicine* and a reoccurring time by normalizing *morning* to the time period 0800-1200 and *for five days* to occur on the date starting with the next morning from the anchor date and for each of the next four days.

In parallel, the record **18** itself may also categorized according to its type from a finite set of predetermined record types, such as medical report, treatment, prescription, etc. The document type may be available as metadata, e.g., added manually by a practitioner or may be identified automatically using text processing / machine learning techniques such as topic-based classifiers (e.g. PLSA) or support vector machine (SVM) classifiers pre-trained on Bag-of-Words representations or other statistically-based representations of labeled records. The record type may be used in determining rules for extraction of the action to be performed, temporal information, and/or other information used in generating the candidate calendar entry.

In some embodiments, the record may be in a structured or semi-structured format which allows the temporal information and/or action, and optionally other information, to be extracted from predefined fields of the record.

Then, using the record type and the context of the temporal expression, the analysis component **20** may label the following identified cases:
1. Clearly defined appointments (points) with an exact date in the future. These may be directly added to the electronic calendar or used to generate a candidate calendar entry which specifies the exact date. If information is available about the healthcare practitioner with whom the appointment is to be held, such as the doctor's name, and/or the location of the laboratory or healthcare practitioner where the appointment is to take place, or is detected with a corresponding Name Entity detector, this can also be added to the calendar, in appropriate healthcare practitioner and location fields **104, 106.**
2. Required appointments with vague date in the future (genpoints) such as "need a further checking in the next two months", or "The next echography should be done in about 2 months." The system may add such items to the "Tasks" in the calendar and/or generate candidate calendar entries in the form of tentative reminders for the user to make these appointments. Again related information (the practitioner's address, phone, the location and contact information of the suggested laboratory) may be added as extra information.
3. For recurring events with precise dates and duration, such as medication (e.g., 3 times a day during 5 days), insulin before meals, half hour physiotherapy sessions twice a week for six weeks, the system may decompose the set of events into individual ones before entering them in the calendar or use the calendar's "recurring appointments" functionality directly.

In each case, the interface generator **22** may display the proposed entries to the user via the GUI **40.**

### B. Processing records corresponding to a change in the health condition

Some changes in the condition of a person may entail setting in place a long-term medical protocol, e.g., over few months or years. For example, as soon as a woman's pregnancy is diagnosed, the calendar update component **26** may add or the interface generator suggest to add several appointments in her calendar according to a stored protocol, for example, for scheduling:
1. one or more pregnancy ultrasounds,
2. regular doctor's appointments,
3. one or more toxoplasmosis screenings depending on the information which recorded in the PHR, etc.

The date suggestions for such appointments can be based on external knowledge, e.g., some actions may be recommended by protocols or mandatory by law. For example, three pregnancy ultrasounds are mandatory in France, specifically, between the 9th and 14th weeks, between the 20th and the 22nd weeks, and between the 32nd and 34th weeks.

### C. Processing contextual information to identify events

Some actions may be triggered, not based on the addition of new medical records, but on "contextual" information, such as the (i) patient's age, (ii) the time of the year or (iii) prior information about the health condition in the family (e.g., information that one of the patient's parents died of cancer). Here are three examples:
1. Vaccines have to be first administered at a given age. Some of the vaccines have then to be re-administered regularly every few years.
2. For young children and elderly people, it is strongly advised to get the flu vaccine during the fall season.
3. After a certain age, patients should obtain regular laboratory tests to diagnose diseases such as cancer, especially if relatives were diagnosed with such diseases in the past.

The MET system may include a set of rules for implementing the generation of context-based appointments. The context-based information used for generating the appointments is regularly checked/matched with the patient information (e.g., age) and the MET system may suggest corresponding actions when certain conditions are met.

For example, rules could express:
if DATE is between 09-01-YYYY and 12-31-YYYY and flu vaccine is not scheduled, generate vaccine scheduling appointment, or
if AGE is greater than 40 and Family History includes (colon) cancer death record, and if no (colon) cancer screening has been recorded in patient's history in last 5 years, then generate (colon) cancer screening scheduling appointment.

### Interaction with User (S108-S112)

In the exemplary embodiment, the appointments and other actions which are suggested by the MET system **10** are validated by the user before they are added to the PEC (in the same way a meeting request needs to be validated by a user before it is permanently added to a PEC). This is to avoid the risk that the MET system may not deliver 100% accuracy. Therefore, presenting the proposed calendar entries to the user allows the user to correct potential mistakes.

As an example, the following cases are considered:
1. The candidate calendar entry is correct and the user (e.g., individual or person acting on behalf of the individual) can accept it as is.
2. The candidate calendar entry is partially correct and the user can correct it before accepting it.
3. The candidate calendar entry is incorrect, in which case the user can delete it.

The candidate calendar entry can be generated to appear automatically on the display of the individual's (or caregiver's) personal computing device, for example, in a similar manner to pop-up reminders that appear when the user's calendar application is running. The user may ignore the candidate calendar entries if it is not convenient to review them at that time and retrieve them later by clicking on a reminder icon. In some embodiments, the interface generator **22** may be configured to generate an alarm with the candidate calendar entry, such as a sound or vibration of the user's mobile device. This may be limited to cases in which the analysis component **20** determines that the candidate calendar entry is of a higher importance than other candidate calendar entries, for example, if the event relates to a medication that should be taken before a specified time and that time has passed.

To help the user decide whether the candidate calendar entry **24** is correct or not, the record (or a piece of the record) **18** that was used to identify the event **86** and time(s) **88, 90** proposed in the candidate calendar entry may be attached to the candidate calendar entry, e.g., in record field **94,** or as a link. The relevant information **108** used to generate the information added to the various fields may be highlighted, as shown in FIGURE 3.

On average, it is easier and faster for a patient to correct an appointment **24** than to enter it from scratch. There may be cases where it is more time consuming for the patient to correct the appointment, for example, if most of the prefilled information is incorrect. However, the cases where few or no changes are needed make the overall average time lower than when entering appointments without the system **10.** Additionally, due to recent standardization of medical records, structured representations are now often present in PHRs, which can facilitate the automatic analysis of records and allow for the use of rules for processing a given field of a record to generate an entry in a corresponding field of the candidate calendar entry form **82.**

The MET system can also be used to propose an acceptable time in the calendar **14** to book an appointment / reminder which is within a larger time-frame identified from the record or to propose a different time if the identified time clashes with another appointment in the user's calendar. As examples:
1. For recurring medication, the proposed time **88, 90** at which the medication is taken can be a combination of the doctor's recommendation plus the patient's personal information, such as: times at which a patient generally takes meals, hour at which children go to be bed, and the like, which may be retrieved from the ODL **72** and/or PEC **14.** For example, the user's calendar may exclude the time period from 8 am to 9 am, in which case, for a prescription to take the medicine in the morning, the time proposed may be 7-7.30 am or 9.15-9.30 am.
2. The system may be used to re-schedule an already defined appointment based on information in a user's calendar and doctor's / laboratory's calendars.
3. For events which have a vague date, the system may be used to propose various dates until a defined date is accepted.

In some embodiments, the MET system **10** may be authorized access to several PHRs **12,** i.e., not only the user's PHR. This may be advantageous in the case of parents or other caregivers wishing to receive the related information (appointments, medications) from their children's medical records and/or those of their elderly family members. For example, a caregiver with responsibility for several patients may employ an MET system **10** which has access to each of their PHR's **12** so that calendar entries generated from each can be entered in the caregiver's PEC **14** and not conflict with each other. In these cases, the validation may be performed by the patient or the caregiver, and the calendar entry may be entered into both their calendars. If both the patient and caregiver have connected the MET system to their PEC, the MET system may access both calendars so that the time proposed is acceptable to both.

A practitioner MET system may be configured for healthcare practitioners and caregivers to access only the relevant information from the PHRs of their regular patients, e.g., in the case of a caregiver working in a nursing home or who routinely visiting patients at their residences, etc. The practitioner MET may have access to only certain types of records in the PHR and thus propose calendar entries only in relation to those records.

The MET system **10** may further identify contact information related to practitioners such as e-mail, phone number, and address, and add the contact information to the user's contacts or update them if appropriate. When the MET system sends a reminder to the PEC, it can then also display the practitioner's information, e.g., the phone number when the appointment has not yet been taken, or the address, and a proposed itinerary to get to the practitioner's office once it is time to go for the appointment.

Where the caregiver has authorization to receive candidate calendar entries for two or more individuals, such as a parent with a child or children, the accepted calendar entry is entered by the update component **26** on the user's calendar clearly identifying the individual to which it refers. In some embodiments, the user may have access to several calendars on which the calendar entries for respective individual are entered. In some embodiments, the medical-related entries on the individual's calendars may be merged into the user's calendar.

It will be appreciated that variants of the above-disclosed and other features and functions, or alternatives thereof, may be combined into many other different systems or applications. Various presently unforeseen or unanticipated alternatives, modifications, variations or improvements therein may be subsequently made by those skilled in the art which are also intended to be encompassed by the following claims.

## Claims

1. A medical event tracking system comprising:
memory which stores instructions for:
detecting new records in a personal health record of a single individual,
identifying one of the new records that refers to an event for which an action is to be performed by at least one of the individual and a person acting on behalf of the individual,
generating a candidate calendar entry for the identified new record which proposes a time for the action to be performed by the at least one of the individual and the person acting on behalf of the individual,
providing for receiving validation of the candidate calendar entry, and
providing for sending the validated candidate calendar entry to a personal electronic calendar of at least one of the individual and the person acting on behalf of the individual; and
a processor in communication with the memory which executes the instructions.

2. The system of claim 1, wherein the identifying one of the new records that refers to an event for which an action is to be performed comprises natural language processing text of the record including applying a set of rules for detecting events in the text.

3. The system of claim 1, wherein the identifying one of the new records that refers to an event for which an action is to be performed comprises providing for detection of temporal expressions in text of the record, and wherein the generating of the candidate calendar entry comprises proposing the time for the action to be performed based on a detected temporal expression.

4. The system of claim 1, wherein the providing for receiving validation of the candidate calendar entry comprises providing for the individual or the person acting on behalf of the individual to perform one of: accepting the candidate calendar entry without modification, making modifications to the candidate calendar entry, and declining to validate the candidate calendar entry.

5. The system of claim 1, wherein the generating of the candidate calendar entry for the identified new record comprises proposing multiple appointments when a recurring event is detected in the new record.

6. The system of claim 1, wherein the generating of the candidate calendar entry for the identified new record comprises incorporating at least a portion of the identified new record into the candidate calendar entry.

7. The system of claim 1, wherein the medical event tracking system is configured for generating candidate calendar entries from records comprising:
(i) records containing temporal information;
(ii) records corresponding to changes in a medical condition; and
(iii) records containing contextual information selected from the individual's age, a time of year, and family medical history.

8. The system of claim 1, wherein the personal health record of the individual includes a plurality of records selected from the group consisting of:
personal medical history records,
family medical history records,
records of test results;
records of prescriptions;
records of clinical data;
narrative doctor notes; and
observations of Daily Living.

9. The system of claim 1, further comprising a display device and wherein the instructions comprise instructions for displaying the candidate calendar entry on the display device orwherein when the event for which an action is to be performed is an appointment with a healthcare practitioner, the generating a candidate calendar entry comprises extracting at least one of healthcare practitioner information and location information from the record and proposing a time for the appointment with the healthcare practitioner or at the location.

10. A portable computing device comprising:
a display device;
a user input device;
a processor; and
memory which stores:
a personal health record of a single individual which includes a set of records pertaining to the health of the individual,
a personal electronic calendar for the individual; and
instructions for:
checking for new records which have been added to the personal health record,
processing the new records to identify a new record which refers to an event for which an action is to be performed by the individual,
generating a candidate calendar entry for the identified new record which proposes a time for the action to be performed by the individual,
displaying the candidate calendar entry in a user interface on the display device;
providing for validation of the candidate calendar entry by the individual via the user input device;
if the candidate calendar entry is validated, sending the validated candidate calendar entry to the personal electronic calendar.
